# EUROPEAN PATENT SPECIFICATION

(11) **EP 1 220 956 B1**
(45) Date of publication and mention of the grant of the patent: **02.07.2003**
(21) Application number: 00969362.3
(22) Date of filing: 27.09.2000
(51) Int. Cl.: C23F 11/12, C23F 11/14, A61K 7/00, A61K 7/06

(54) **CORROSION INHIBITING ADDITIVE FOR COSMETIC PRODUCTS**
KORROSIONSHEMMENDER ZUSATZ FÜR KOSMETISCHE PRODUKTE
ADDITIF INHIBANT LA CORROSION POUR PRODUITS COSMETIQUES

(30) Priority: 14.10.1999 GB 9924370
(43) Date of publication of application: 10.07.2002
(73) Proprietor: UNILEVER PLC, London EC4P 4BQ (GB); UNILEVER N.V., 3013 AL Rotterdam (NL)
(72) Inventor: GRIFFITHS, Llyr Glyndwr, Wirral, Merseyside L62 4ZD (GB); HAYE, Frederic, Elida Faberge, F-60881 Le Meux Cedex (FR)
(74) Representative: Tansley, Sally Elizabeth
(86) International application number: EP0009591
(87) International publication number: WO01027351

(56) References cited:
- WO-A-95/14528
- US-A- 3 878 938
- US-A- 5 328 635
- US-A- 5 348 731

## Description

### FIELD OF THE INVENTION

The invention concerns sprayable hair treatment products, such as hair styling mousses and mousse shampoos, dispensed from pressurisable metal cans.

### BACKGROUND AND PRIOR ART

Spray cans such as pumps or aerosols are widely used as delivery systems for hair treatment products. The problem of can corrosion is becoming particularly apparent as the transition to water-based formulations takes place for cost and environmental reasons.

In an attempt to overcome this problem, aluminium cans have been employed. Unfortunately, these materials are not as economical as ordinary tin-plated steel. Furthermore, aluminium cans can also be susceptible to corrosion.

Corrosion is manifest as, for example, unsightly rust and, for aerosol formulations, loss of propellant. Moreover, oxidation can transform formulation components, such as those constituting the fragrance, with consequential adverse effects on odour. When corrosion is severe enough it may ultimately cause leaking of the can in addition to contamination of the aerosol product inside.

In tin-plated cans, some amount of protection against corrosion is afforded by the tin, especially in the absence of any significant amount of water. However, product forms such as mousses, e.g. styling mousses or aerosol shampoos will typically contain a significant amount of water. Furthermore, even for systems traditionally based on organic solvents rather than water, such as hairsprays, environmental concerns and cost considerations have forced replacement of the organic solvent with, at least in part, water.

Furthermore, pressurised cans require valves, which are inherently more susceptible to corrosion than the body of the can itself, since the valve material is necessarily highly stressed during the process of assembly, making it a focus for corrosive attack.

As described in Boulden, *"Corrosion Inhibitors for Water-Based Aerosol Formulations",* Spray Technology and Marketing April 1993, the problem of corrosion has been a barrier to the introduction of many water-based aerosol formulations, even in aluminium containers, and there is no generally applicable solution to this problem.

Rocafort, *"Preventing Corrosion in Aqueous Based Aerosols",* Spray Technology and Marketing December 1995, outlines the main approaches to minimising corrosion from fluids inside an aerosol container, of which one is the inclusion of corrosion inhibitors. The article describes some typical examples of the different types of corrosion inhibitors (phosphates, silicates, bicarbonates and amines) and gives screening results for a range of inhibitors tested individually. It is mentioned that the "optimum corrosion fix" would involve, *inter alia*, the utilisation of a synergistic combination of inhibitors simultaneously, but there is no specific guidance given on what the chemical constituents of such a combination might be.

Examples of systems which have been found to be effective for corrosion inhibition in hairsprays are ammonium hydroxide, and a combination of sodium benzoate and cyclohexamine. Further examples are described in the following patents:
U.S. Pat. No. 4,604,226 discloses an inhibitor system comprising a mixture of an amine neutralised phosphate ester and a volatile amine, the latter selected from cyclohexamine, morpholine and isopropylamine.
U.S. Pat. No. 4,584,021 describes related technology wherein the inhibitor system is a mixture of nitroalkane and an amine neutralised phosphate ester.
U.S. Pat. No. 4,263,275 reports a hydroalcoholic, pressurised hairspray composition wherein the anticorrosion agent is a phosphate salt of a quaternary ammonium compound.
U.S. Pat. No. 5,348,731 discloses ammonium benzoate as an effective corrosion inhibitor for steel can packages containing sprayable hair treatment products, particularly hairsprays. The ammonium benzoate is preferably employed in combination with ammonium hydroxide, since this combination is said to deliver more than just an additive effect.
US Pat. No. 4,673,569 describes a mousse hair product which comprises a long chain nonionic ester which is said to reduce corrosivity when used in combination with corrosion inhibitors.
US Pat. No. 5,462,727 describes an aqueous hair spray composition having a corrosion inhibitor consisting essentially of 0.05-5% of an alkyl benzoate and 0.01-5% of an organic borate.
WO 95/14528 discloses the use of certain bis-oxazolidine compounds as corrosion inhibitors.

We have now found that a combination of a benzoate salt and an oxazolidine provides exceptionally effective corrosion inhibition. Surprisingly, the combination delivers more than just an additive effect.

### SUMMARY OF THE INVENTION

The present invention provides a personal care composition suitable for packaging in a metal can, the composition including a corrosion-inhibiting additive which is a combination of a benzoate salt and an oxazolidine.

### DETAILED DESCRIPTION

### Benzoate Salt

Examples of suitable benzoate salts according to the invention include alkali metal, alkaline earth metal and ammonium salts, and mixtures thereof. Sodium benzoate is particularly preferred.

Amounts of the benzoate salt may range from 0.01 to 5%, preferably from 0.05 to 2%, optimally from 0.1 to 1% by weight based on total weight of the personal care composition.

### Oxazolidine

Compositions of the present invention contain a further corrosion inhibitor in the form of an oxazolidine. Oxazolidines are produced when aldehydes are reacted with primary amino alcohols.

Examples of suitable oxazolidines according to the invention include monocyclic oxazolidines such as 4,4-dimethyl-1,3-oxazolidine and 3,4,4-trimethyl-1,3-oxazolidine, bicyclic oxazolidines such as 1-aza-3,7-dioxa-5-ethylbicyclo (3.3.0) octane, and mixtures thereof. Particularly preferred is 4,4-dimethyl-1,3-oxazolidine.

The above materials are all available commercially from ANGUS Chemie GmbH under the tradenames OXABAN®-A, BIOBAN® CS-1135, OXABAN®-E and BIOBAN® CS-1246. Particularly preferred is OXABAN®-A, which is an aqueous solution of 4,4-dimethyl-1,3-oxazolidine.

Amounts of the oxazolidine may range from 0.001 to 5%, preferably from 0.005 to 1%, optimally from 0.05 to 0.2% by weight based on total weight of the personal care composition.

### Product Form

Compositions of the present invention can be formulated into a wide variety of product types, including mousses, gels, lotions, tonics, sprays, shampoos, conditioners, rinses, hand and body lotions, facial moisturisers, sunscreens, anti-acne preparations, shaving foams, topical analgesics, mascaras, and the like.

### Shampoo Compositions

The corrosion-inhibiting additive of the present invention is particularly useful with shampoo compositions.

Such compositions are generally characterised by containing at least one surfactant, to provide a cleansing benefit. Surfactant may also be present as emulsifier for emulsified conditioning agents which may be present in the shampoo, such as silicones.

Further surfactant(s) will be present as an additional cleansing ingredient if sufficient for cleansing purposes is not provided as the emulsifier for the emulsified conditioning agents. This further cleansing surfactant may be the same surfactant as the emulsifier, or may be different.

Suitable emulsifiers are well known in the art and include anionic and nonionic surfactants. Examples of anionic surfactants used as emulsifiers are alkylarylsulphonates, e.g., sodium dodecylbenzene sulphonate, alkyl sulphates e.g., sodium lauryl sulphate, alkyl ether sulphates, e.g., sodium lauryl ether sulphate nEO, where n is from 1 to 20 alkylphenol ether sulphates, e.g., octylphenol ether sulphate nEO where n is from 1 to 20, and sulphosuccinates, e.g., sodium dioctylsulphosuccinate.

Examples of nonionic surfactants used as emulsifiers are alkylphenol ethoxylates, e.g., nonylphenol ethoxylate nEO, where n is from 1 to 50, alcohol ethoxylates, e.g., lauryl alcohol nEO, where n is from 1 to 50, ester ethoxylates, e.g., polyoxyethylene monostearate where the number of oxyethylene units is from 1 to 30.

Cleansing surfactants are typically selected from anionic, nonionic, amphoteric and zwitterionic surfactants, and mixtures thereof.

Suitable anionic cleansing surfactants for compositions of the invention include the alkyl sulphates, alkyl ether sulphates, alkaryl sulphonates, alkanoyl isethionates, alkyl succinates, alkyl sulphosuccinates, N-alkoyl sarcosinates, alkyl phosphates, alkyl ether phosphates, alkyl ether carboxylates, alpha-olefin sulphonates and acyl methyl taurates, especially their sodium, magnesium ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from one to 10 ethylene oxide or propylene oxide units per molecule, and preferably contain 2 to 3 ethylene oxide units per molecule.

Examples of suitable anionics include sodium lauryl sulphate, sodium lauryl ether sulphate, ammonium lauryl sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauroyl isethionate, and sodium N-lauryl sarcosinate.

Nonionic cleansing surfactants suitable for use in shampoo compositions of the invention may include condensation products of aliphatic (C₈-C₁₈) primary or secondary linear or branched chain alcohols or phenols with alkylene oxides, usually ethylene oxide and generally having from 6 to 30 ethylene oxide groups. Other suitable nonionics include alkylpolyglycosides and mono- or di-alkyl alkanolamides.

Examples of the latter nonionics include coco mono- or diethanolamide and coco mono-isopropanolamide.

Amphoteric and zwitterionic cleansing surfactants suitable for use in compositions of the invention may include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates and alkyl amidopropyl hydroxysultaines. Examples include lauryl amine oxide, cocodimethyl sulphopropyl betaine and preferably lauryl betaine, cocamidopropyl betaine and sodium cocamphopropionate.

The total amount of surfactant (including any used as emulsifier for the conditioning agent) is generally from 1 to 50%, preferably from 5 to 30%, more preferably from 10% to 25%, by total weight of surfactant based on total weight of the shampoo composition.

The shampoo composition will typically comprise a predominantly aqueous carrier, water forming the continuous phase in which any particles of water-insoluble conditioning agent are dispersed. Water is generally present in an amount of from 10 to 99%, preferably from 20 to 80%, more preferably from 40 to 75%, by total weight of water based on total weight of the shampoo composition.

A particularly preferred product form according to the invention is an aerosol shampoo mousse based on surfactant and water as described above and further containing an aerosol propellant. This agent is responsible for expelling the other materials from the container and forming the mousse character.

The propellant gas can be any liquefiable gas conventionally used for aerosol containers. Examples of suitable propellants include dimethyl ether, propane, n-butane and isobutane, used singly or admixed. Other examples of propellants are nitrogen, carbon dioxide, compressed air and fluorohydrocarbons such as the material sold by Du Pont under the trade name DYMEL® 152a.

The amount of the propellant gases is governed by normal factors well known in the aerosol art. For mousses the level of propellant is generally from about 1 to about 15%, optimally from about 2 to about 10% for creamy foam and good sensory feel.

### Hair Styling Compositions

The corrosion-inhibiting additive of the present invention is also particularly useful with hair styling compositions. These compositions are characterised by containing at least one hair styling polymer.

Hair styling polymers are well known articles of commerce and many such polymers are available commercially which contain moieties which render the polymers cationic, anionic, amphoteric or nonionic in nature. The polymers may be synthetic or naturally derived.

The amount of the polymer may range from 0.5 to 10%, preferably 0.75 to 6% by weight of the total composition.

Examples of anionic hair styling polymers are:
copolymers of vinyl acetate and crotonic acid;
terpolymers of vinyl acetate, crotonic acid and a vinyl ester of an alpha-branched saturated aliphatic monocarboxylic acid such as vinyl neodecanoate;
copolymers of methyl vinyl ether and maleic anhydride (molar ratio about 1:1) wherein such copolymers are 50% esterified with a saturated alcohol containing from 1 to 4 carbon atoms such as ethanol or butanol;
acrylic copolymers containing acrylic acid or methacrylic acid as the anionic radical-containing moiety with other monomers such as: esters of acrylic or methacrylic acid with one or more saturated alcohols having from 1 to 22 carbon atoms (such as methyl methacrylate, ethyl acrylate, ethyl methacrylate, n-butyl acrylate, t-butyl acrylate, t-butyl methacrylate, n-butyl methacrylate, n-hexyl acrylate, n-octyl acrylate, lauryl methacrylate and behenyl acrylate); glycols having from 1 to 6 carbon atoms (such as hydroxypropyl methacrylate and hydroxyethyl acrylate); styrene; vinyl caprolactam; vinyl acetate; acrylamide; alkyl acrylamides and methacrylamides having 1 to 8 carbon atoms in the alkyl group (such as methacrylamide, t-butyl acrylamide and n-octyl acrylamide); and other compatible unsaturated monomers.

The polymer may also contain grafted silicone, such as polydimethylsiloxane.

Specific examples of suitable anionic hair styling polymers are:
RESYN® 28-2930 available from National Starch (vinyl acetate/crotonic acid/vinyl neodecanoate copolymer);
ULTRAHOLD® 8 available from BASF (CTFA designation Acrylates/acrylamide copolymer);
the GANTREZ® ES series available from ISP Corporation esterified copolymers of methyl vinyl ether and maleic anhydride).

Other suitable anionic hair styling polymers include carboxylated polyurethanes. Carboxylated polyurethane resins are linear, hydroxyl-terminated copolymers having pendant carboxyl groups. They may be ethoxylated and/or propoxylated at least at one terminal end. The carboxyl group can be a carboxylic acid group or an ester group, wherein the alkyl moiety of the ester group contains one to three carbon atoms. The carboxylated polyurethane resin can also be a copolymer of polyvinylpyrrolidone and polyurethane, having a CTFA designation PVP/polycarbamyl polyglycol ester. Suitable carboxylated polyurethane resins are disclosed in EP 0 619 111 A1 and US Patent No. 5,000,955. Other suitable hydrophilic polyurethanes are disclosed in US Patent Nos. 3,822,238; 4,156,066; 4,156,067; 4,255,550; and 4,743,673.

Amphoteric polymers which can contain cationic groups derived from monomers such as t-butyl aminoethyl methacrylate as well as carboxyl groups derived from monomers such as acrylic acid or methacrylic acid can also be used in the present invention. One specific example of an amphoteric hair styling polymer is Amphomer® (Octylacrylamide/acrylates/butylaminoethyl methacrylate copolymer) sold by the National Starch and Chemical Corporation.

Examples of nonionic hair styling polymers are homopolymers of N- vinylpyrrolidone and copolymers of N-vinylpyrrolidone with compatible nonionic monomers such as vinyl acetate. Nonionic polymers containing N- vinylpyrrolidone in various weight average molecular weights are available commercially from ISP Corporation - specific examples of such materials are homopolymers of N-vinylpyrrolidone having an average molecular weight of about 630,000 sold under the name PVP K-90 and are homopolymers of N-vinylpyrrolidone having an average molecular weight of about 1,000,000 sold under the name of PVP K-120.

Other suitable nonionic hair styling polymers are cross-linked silicone resins or gums. Specific examples include rigid silicone polymers such as those described in EP-A-240 350 and cross-linked silicone gums such as those described in WO 96/31188.

Examples of cationic hair styling polymers are copolymers of amino-functional acrylate monomers such as lower alkyl aminoalkyl acrylate, or methacrylate monomers such as dimethylaminoethyl methacrylate, with compatible monomers such as N-vinylpyrrolidone, vinyl caprolactam, alkyl methacrylates (such as methyl methacrylate and ethyl methacrylate) and alkyl acrylates (such as ethyl acrylate and n-butyl acrylate).

Specific examples of suitable cationic polymers are:
copolymers of N-vinylpyrrolidone and dimethylaminoethyl methacrylate, available from ISP Corporation as Copolymer 845, Copolymer 937 and Copolymer 958;
copolymers of N-vinylpyrrolidone and dimethylaminopropylacrylamide or methacrylamide, available from ISP Corporation as Styleze® CC10;
copolymers of N-vinylpyrrolidine and dimethylaminoethyl methacrylate;
copolymers of vinylcaprolactam, N-vinylpyrrolidone and dimethylaminoethylmethacrylate;
Polyquaternium-4 (a copolymer of diallyldimonium chloride and hydroxyethylcellulose);
Polyquaternium-11 (formed by the reaction of diethyl sulphate and a copolymer of vinyl pyrrolidone and dimethyl aminoethylmethacrylate), available from ISP as Gafquat® 734, 755 and 755N, and from BASF as Luviquat® PQ11;
Polyquaternium-16 (formed from methylvinylimidazolium chloride and vinylpyrrolidone), available from BASF as Luviquat®FC 370, FC 550, FC 905 and HM-552;

Polyquaternium-46 (prepared by the reaction of vinylcaprolactam and vinylpyrrolidone with methylvinylimidazolium methosulphate), available from BASF as Luviquat®Hold.

Examples of suitable naturally derived polymers include shellac, alginates, gelatins, pectins, starch, cellulose derivatives and chitosan or salts and derivatives thereof. Commercially available examples include Kytamer® (ex Amerchol) and Amaze® (ex National Starch).

With certain of the above-described polymers it may be necessary to neutralise some acidic groups to promote solubility/dispersibility. Examples of suitable neutralising agents include 2-amino-2- methyl-1, 3-propanediol (AMPD); 2-amino-2-ethyl-1,3-propanediol (AEPD); 2-amino-2-methyl-1-propanol (AMP); 2-amino-1-butanol (AB); monoethanolamine (MEA); diethanolamine (DEA); triethanolamine (TEA); monoisopropanolamine (MIPA); diisopropanol-amine (DIPA); triisopropanolamine (TIPA); and dimethyl stearamine (DMS). A long chain amine neutralising agent such as stearamidopropyl dimethylamine or lauramidopropyl dimethylamine may be employed, as is described in US 4,874,604. Also suitable are inorganic neutralisers, examples of which include sodium hydroxide, potassium hydroxide and borax. Mixtures of any of the above neutralising agents may be used. Amounts of the neutralising agents will range from about 0.001 to about 10% by weight of the total composition.

Hair styling compositions are typically formulated as sprays, mousses, lotions or gels and can be in aerosol or nonaerosol forms. If an aerosol product form is desired, a propellant must be included in the composition. Suitable propellants are described above in the context of aerosol shampoo mousses.

For hairsprays the level of propellant is generally from 3 to 80%, preferably from 5 to 60%, optimally from 30% to 50% by weight of propellant based on total weight of the hairspray.

### Other Ingredients

Compositions according to the present invention may contain any other ingredient normally used in personal care compositions, and depending on the intended use of the composition. These other ingredients may include bacteriostats (such as triclosan) for deodorants, perspiration inhibitors (such as aluminium or zirconium salts) for antiperspirants, conditioning agents (such as emollients, lubricants and moisturisers), for skin or hair conditioning products such as post-wash hair conditioners, hair or body conditioning shampoos and shaving foams, colouring agents, antifoam agents, antioxidants, fragrances, antimicrobials, solvents for components such as hair styling polymers, and sunscreens. Each of these ingredients will be present in an amount effective to accomplish its purpose.

Examples of preferred optional ingredients in compositions of this invention are adjuvants suitable for hair care. Generally such ingredients are included individually at a level of up to 2%, preferably up to 1%, by weight of the total composition.

Among suitable hair care adjuvants, are:
(i) natural hair root nutrients, such as amino acids, sugars and vitamins. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine.
(ii) hair fibre benefit agents. Examples are:
   ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.
   fatty acids, for cuticle repair and damage prevention. Examples are branched chain fatty acids such as 18-methyleicosanoic acid and other homologues of this series, straight chain fatty acids such as stearic, myristic and palmitic acids, and unsaturated fatty acids such as oleic acid, linoleic acid, linolenic acid and arachidonic acid. A preferred fatty acid is oleic acid. The fatty acids may be added singly, as mixtures, or in the form of blends derived from extracts of, e.g. lanolin.

Mixtures of any of the above active ingredients may also be used.

The invention is further illustrated by way of the following non-limitative examples, in which all percentages quoted are by weight based on total weight unless otherwise stated:

### Packaging

Compositions according to the present invention are suitable for packaging in containers made from any material, especially aluminium, tin-plate, and including mixtures or other combinations of these. Tin-plate is particularly preferred for aerosol cans for cost reasons. Preferably, for metal containers, the interior surface of the metal container (and the valve, in the case of pressurised metal cans provided with such) is laminated with a plastic material or coated with a lacquer or with a varnish, to further protect the interior surface from corrosion. Preferred materials for lamination and lacquers or varnishes for coating are Micoflex®, epoxyphenolic, PAM, PET, PP, Ferrolite® or a combination thereof.

The invention will now be further illustrated by the following, non-limiting Examples:

### EXAMPLES

### Control Formulation

A mousse shampoo base was made up having the following ingredients:

| **Ingredient** | **% (wt)** |
|---|---|
| Sodium laureth sulphate | 17.143 |
| Cocamidopropyl betaine | 6.67 |
| Sodium cocoyl isethionate | 2.000 |
| Silicone Emulsion ex Dow Corning (dimethiconol in anionic surfactant, 25% a.i.) | 6.0000 |
| Sodium benzoate | 0.5000 |
| DMDM Hydantoin, Iodopropynyl butylcarbamate | 0.2000 |
| Guar hydroxypropyltrimonium chloride | 0.1000 |
| Perfume | 0.5000 |
| PPG-9 | 0.0010 |
| Water, minors | q.s. |

### Corrosion Testing

Formulations were made up from the control formulation described above, using various corrosion inhibitors, as follows:

| **Test Formula** | **Formulation** |
|---|---|
| Control | Control formulation only |
| A | Control formulation + 1.0% CRODASINIC® LS30 (Sodium lauryl sarcosinate, ex Croda Chemicals Ltd) |
| B | Control formulation + 1.0% CRODASINIC® O (Oleoyl sarcosine, ex Croda Chemicals Ltd) |
| C | Control formulation + 1.0% DEHYQUART® SP (Quaternium-52, ex Henkel) |
| D | Control formulation + 1.0% WITCONOL® 14 (Polyglyceryl-4 oleate, ex Witco) |
| E | Control formulation + 1.0% OXABAN®A (Dimethyl oxazolidone ex Angus Chemie GmbH) |

The corrosion test involved immersing a valve cup in the test formula and storage testing at 50°C. The valve cup was tin-plate having an epoxyphenolic external lacquer and a 200 micron PP laminate on the interior.

The results showed significant corrosion of the valve cup after 2 weeks storage for all the test formulas except test formula E. The valve cup immersed in test formula E showed no signs of corrosion even after 5 weeks storage under the same conditions.

A further corrosion test was conducted using the control and test formula E. Steel-based can components having various types of coating were immersed in the test formula and soaked for 3 months at room temperature. The results are shown in the following table:

| Coating on can component | Corrosion observed after 3 months | |
|---|---|---|
| | with Control | with Test Formula E |
| PP 40 micron | 4 | 5 |
| PP 25 micron | 4 | 5 |
| PP 15 micron | 4 | 5 |
| Clear PET 23 micron | 4 | 5 |
| White PET 23 micron | 4 | 5 |
| 5 micron uncoloured epoxy | 3 | 5 |
| 5 micron black adhesive + PET 20 micron | 1 | 5 |

### Coding for corrosion results:

| | |
|---|---|
| 5 | Perfect - no visible change |
| 4 | Some colour change apparent |
| 3 | Marked colour change apparent |
| 2 | Visible points of corrosion |
| 1 | Obvious corrosion |

It was noted that in all the tests using the control, the formula became discoloured (rust coloured) and lost viscosity, whereas in all the tests using test formula E, the test formula did not show discoloration and maintained viscosity.

A third test was conducted using a pressurised system. A tin-plate can with a PET laminate body and Ferrolite® components was filled with test formula and charged with propellant (4.0% Drivosol® 2.7, ex Huls). The test formulas used were the control and a test formula F, corresponding to the control formula with the addition of 0.10% OXABAN®A.

The cans were stored for 3 months in a horizontal position at 37°C and 50°C respectively. After this period, visual signs of corrosion were seen in one of the components in the presence of the control formula. In contrast, no such change was evident in the presence of test formula F.

## Claims

1. A personal care composition suitable for packaging in a metal can, the composition including a corrosion-inhibiting additive which is a combination of a benzoate salt and an oxazolidine.

2. A composition according to claim 1, in which the level of benzoate salt ranges from 0.01 to 5% by weight based on total weight of the composition.

3. A composition according to claim 1 or 2, in which the benzoate salt is sodium benzoate.

4. A composition according to any one of claims 1 to 3, in which the level of oxazolidine ranges from 0.001 to 5% by weight based on total weight of the composition.

5. A composition according to any one of claims 1 to 4, in which the oxazolidine is 4,4-dimethyl-1,3-oxazolidine.

6. A composition according to any of claims 1 to 5, which is in the form of an aerosol shampoo mousse comprising from 5 to 30% surfactant, by weight based on total weight, and from 20 to 80% water, by weight based on total weight.

7. A composition according to any of claims 1 to 5, which is in the form of a hair styling composition comprising at least one hair styling polymer.

8. A composition according to any of claims 1 to 7, which is packaged in a tin-plate can.

## Patentansprüche

1. Körperpflegezusammensetzung, die zum Verpacken in einer Metalldose geeignet ist, wobei die Zusammensetzung einen korrosionshemmenden Zusatz einschließt, der eine Kombination eines Benzoatsalzes und eines Oxazolidins darstellt.

2. Zusammensetzung nach Anspruch 1, worin der Anteil des Benzoatsalzes im Bereich von 0,01 bis 5 Gewichtsprozent, bezogen auf das Gesamtgewicht der Zusammensetzung, ist.

3. Zusammensetzung nach Anspruch 1 oder 2, worin das Benzoatsalz Natriumbenzoat ist.

4. Zusammensetzung nach einem der Ansprüche 1 bis 3, worin der Anteil von Oxazolidin im Bereich von 0,001 bis 5 Gewichtsprozent, bezogen auf das Gesamtgewicht der Zusammensetzung, ist.

5. Zusammensetzung nach einem der Ansprüche 1 bis 4, worin das Oxazolidin 4,4-Dimethyl-1,3-oxazolidin ist.

6. Zusammensetzung nach einem der Ansprüche 1 bis 5, die in Form eines Aerosol-Shampoomousse vorliegt, umfassend 5 bis 30% Tensid, bezogen auf das Gesamtgewicht, und 20 bis 80 Gewichtsprozent Wasser, bezogen auf das Gesamtgewicht.

7. Zusammensetzung nach einem der Ansprüche 1 bis 5, die in Form einer Haarstylingzusammensetzung vorliegt, umfassend mindestens ein Haarstylingpolymer.

8. Zusammensetzung nach einem der Ansprüche 1 bis 7, die in einer Weißblechdose verpackt ist.

## Revendications

1. Composition de soin personnel appropriée pour être conditionnée dans une boîte en métal, la composition incluant un additif inhibant la corrosion qui est une combinaison de sel de benzoate et d'une oxazolidine.

2. Composition selon la revendication 1, dans laquelle le niveau de sel de benzoate est compris dans la gamme allant de 0,01 à 5 % en poids, sur la base du poids total de la composition.

3. Composition selon la revendication 1 ou 2, dans laquelle le sel de benzoate est du benzoate de sodium.

4. Composition selon l'une quelconque des revendications 1 à 3, dans laquelle le niveau d'oxazolidine est compris dans la gamme allant de 0,001 à 5 % en poids sur la base du poids total de la composition.

5. Composition selon l'une quelconque des revendications 1 à 4, dans laquelle l'oxazolidone est de la 4,4 - diméthyle - 1,3 - oxazolidine.

6. Composition selon l'une quelconque des revendications 1 à 5, qui se présente sous la forme d'un shampoing en mousse aérosol comprenant de 5 à 30 % de tensioactif, en poids sur la base du poids total, et de 20 à 80 % d'eau, en poids sur la base du poids total.

7. Composition selon l'une quelconque des revendications 1 à 5, qui est sous la forme d'une composition coiffante pour les cheveux comprenant au moins un polymère de coiffage des cheveux.

8. Composition selon l'une quelconque des revendications 1 à 7, qui est conditionnée dans une boîte en fer-blanc.
